# EUROPEAN PATENT SPECIFICATION

(11) **EP 4 332 581 B1**
(45) Date of publication and mention of the grant of the patent: **16.09.2026**
(21) Application number: 22794204.2
(22) Date of filing: 19.01.2022
(51) Int. Cl.: G01N 35/04, G01N 33/53

(54) **REACTION CUP CONVEYING DEVICE AND IMMUNOLOGICAL DETECTION APPARATUS**
REAKTIONSBECHERFÖRDERVORRICHTUNG UND IMMUNOLOGISCHE NACHWEISVORRICHTUNG
DISPOSITIF DE TRANSPORT DE COUPELLE DE RÉACTION ET APPAREIL DE DÉTECTION IMMUNOLOGIQUE

(30) Priority: 30.04.2021 CN 202110485824
(43) Date of publication of application: 06.03.2024
(73) Proprietor: Shenzhen Yhlo Biotech Co., Ltd, Shenzhen, 518116 Guangdong (CN)
(72) Inventor: HUANG, Xiutao, Shenzhen, Guangdong 518116 (CN); CHEN, Shuxiang, Shenzhen, Guangdong 518116 (CN); ZHANG, Fuxing, Shenzhen, Guangdong 518116 (CN); XIAO, Yujin, Shenzhen, Guangdong 518116 (CN)
(74) Representative: Mewburn Ellis LLP
(86) International application number: PCT/CN2022/072667
(87) International publication number: WO 2022/227722

(56) References cited:
- CN-A- 102 565 439
- CN-A- 109 975 277
- CN-A- 111 521 833
- CN-A- 113 063 957
- CN-U- 206 460 068
- CN-U- 207 557 283
- CN-U- 211 453 664
- CN-U- 214 750 370
- JP-A- H1 019 901
- US-A- 5 244 633
- US-A1- 2020 011 857
- US-B2- 7 273 591

## Description

### TECHNICAL FIELD

The present invention relates to the field of biological detection and diagnosis, and in particular, to a reaction cup conveying device and an immunological detection apparatus.

### BACKGROUND

With the development of modern science, an immunodiagnostic technology has gradually become a key means of modern clinical testing and scientific research. The immunodiagnostic technology is an important diagnostic tool for diseases such as tumors, diabetes, and abnormal secretion of gonads. Compared with conventional detection means, which have disadvantages of complex manual operations, long reaction time, and environmental pollution, the immunodiagnostic technology such as a chemiluminescence immunoassay technology has advantages of short reaction time, simple operations, and high diagnostic efficiency and has been widely used in modern medicine and scientific research fields.

Currently, the chemiluminescent immunoassay system mainly includes: a reaction cup storage and transfer system, a sample loading system, a reagent loading system, a sample adding system, an incubation system, a centrifugal cleaning system, and a luminous reading system. The incubation system is associated with the sample adding system, the centrifugal cleaning system, and the luminous reading system. The incubation system is required to continuously convey a reaction cup to a designated position. In this one-to-many situation, a lot of waiting time is wasted, and efficiency of instrument testing is relatively low. Due to a large number of test items and a large amount of sample testing in large hospitals, the efficiency of instrument testing is far from meeting a requirement.

CN 111 521 833 A discloses a chemiluminescence immunoassay reaction disc with multiple reaction modes and an analyzer capable of meeting multi-mode detection requirements.

### SUMMARY

Accordingly, it is necessary to provide a reaction cup conveying device and an immunological detection apparatus that can implement the accurate conveying of a certain number of reaction cups, and solve the problems of the poor positioning accuracy and complex structure of the conveying of reaction cups by means of an outer annular plate body in an existing mechanism.

Aspects of the invention are set out in the appended claims.

The reaction cup conveying device disclosed herein has the following beneficial effects.

(1) The outer circular conveying plate body of the reaction cup conveying device uses a structure of a gear made of engineering plastics and sleeved with a metal outer plate reinforcing member, which is light and simple in structure and has good gear strength and rigidity, stable power transmission, and durable and accurate positioning accuracy. In use, stability and accuracy of the reaction cup conveying device are guaranteed.

(2) In the above reaction cup conveying device, through the arrangement of the fit rolling member such as the V-shaped roller, the outer edge of the inner circular conveying plate body can be lubricated, so that when rotating at a high speed, the outer circular conveying plate body may not produce high friction noise, rub off powder, or even cause damages to and failure of the roller or the flange with the inner circular conveying plate body. Further, the V-shaped roller may use a self-lubricating material and an oil storage tank structure to prolong the overall service life of the device and reduce the cost of manual maintenance.

(3) The fit rolling member such as the V-shaped roller is in contact with the inner circular conveying plate body by abutment and tangency, i.e., point-to-point contact, which can minimize the contact area between the V-shaped roller and the inner circular conveying plate body, thereby reducing losses of mutual friction and also improving stability of the conveying of the outer circular conveying plate body and positioning accuracy of the conveying. Point-to-point contact friction realizes relative motion, which makes the overall operation of the device more stable, so that the positioning accuracy is more accurate and adaptability is wider.

(4) In the reaction cup conveying device, through the positioning of the V-shaped roller, the outer circular conveying plate body is suspended on the inner circular conveying plate body, the outer circular conveying plate body and the inner circular conveying plate body can convey the reaction cups independently, the outer circular conveying plate body and the inner circular conveying plate body can respectively meet system schemes under different time sequences, the outer circular conveying plate body and the inner circular conveying plate body can operate independently of each other, and positioning parameters can be associated with each other, so application scenarios are more extensive. In addition, the outer circular conveying plate body and the inner circular conveying plate body are positioned through the V-shaped roller, so that the whole device has a simple structure.

### BRIEF DESCRIPTION OF THE DRAWINGS

FIG. 1 is a schematic view of a reaction cup conveying device according to an embodiment of the present invention;

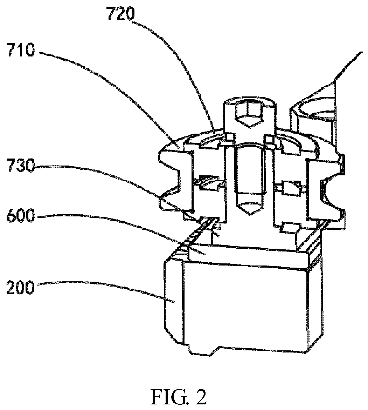

FIG. 2 is a schematic view of a sliding fit mechanism of the reaction cup conveying device according to an embodiment of the present invention; and

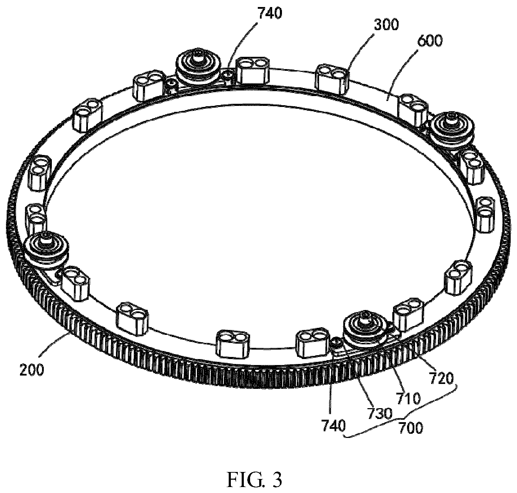

FIG. 3 is a schematic view of an outer circular conveying plate body of the reaction cup conveying device according to an embodiment of the present invention.

### Reference signs

10: reaction cup conveying device; 100: inner circular conveying plate body; 200: outer circular conveying plate body; 300: reaction cup loading assembly; 310: embedding groove; 400: driving mechanism; 500: conveying transmission mechanism; 510: transmission driving gear; 520: transmission shaft; 600: outer plate reinforcing member; 700: sliding fit mechanism; 710: fit rolling member; 720: rolling shaft; 730: bottom plate; 740: fastener.

### DETAILED DESCRIPTION OF THE EMBODIMENTS

In order to make the above objectives, features and advantages of the present invention more obvious and understandable, specific implementations of the present invention are described in detail below with reference to the accompanying drawings. In the following description, many specific details are set forth in order to fully understand the present invention. However, the present invention can be implemented in many other ways different from those described herein, and those skilled in the art can make similar improvements without departing from the connotation of the present invention. Therefore, the present invention is not limited by specific embodiments disclosed below.

In the description of the present invention, it should be understood that the orientation or position relationships indicated by the terms "central", "longitudinal", "transverse", "length", "width", "thickness", "up", "down", "front", "back", "left", "right", "vertical", "horizontal", "top", "bottom", "inner", "outer", "clockwise", "counterclockwise", "axial", "radial", "circumferential", etc. are based on the orientation or position relationships shown in the accompanying drawings and are intended to facilitate the description of the present invention and simplify the description only, rather than indicating or implying that the device or element referred to must have a particular orientation or be constructed and operated in a particular orientation, and therefore cannot be interpreted as limiting the present invention.

In addition, the terms "first" and "second" are used for descriptive purposes only, which cannot be construed as indicating or implying a relative importance or implicitly specifying the number of the indicated technical features. Thus, the features defined with "first" and "second" may explicitly or implicitly include at least one feature. In the description of the present invention, "a plurality of" means at least two, such as two or three, unless specifically stated otherwise.

In the present invention, unless otherwise specifically stated and limited, the terms "install," "join," "connect", "fix", etc. should be understood in a broad sense, such as, a fixed connection, a detachable connection, or an integral connection; a mechanical connection, or an electrical connection; a direct connection, an indirect connection through an intermediate medium, internal communication between two elements, or an interaction of two elements, unless otherwise expressly defined. For those of ordinary skill in the art, the specific meanings of the foregoing terms in the present invention can be understood on a case-by-case basis.

In the present invention, unless otherwise explicitly specified and defined, a first feature being "on" or "under" a second feature may be a case that the first feature is in direct contact with the second feature, or the first feature is in indirect contact with the second feature via an intermediate medium. Furthermore, the first feature being "over", "above" and "on top of" the second feature may be a case that the first feature is directly above or obliquely above the second feature, or only means that the level of the first feature is higher than that of the second feature. The first feature being "below", "underneath" or "under" the second feature may be a case that the first feature is directly underneath or obliquely underneath the second feature, or only means that the level of the first feature is lower than that of the second feature.

It should be noted that when one element is referred to as "fixed to" or "arranged on" another element, it may be directly disposed on another element or an intermediate element may exist. When one element is considered to be "connected to" another element, it may be directly connected to another element or an intermediate element may co-exist. The terms "vertical", "horizontal", "up", "down", "left", "right" and similar expressions used herein are for illustrative purposes only, and do not represent unique implementations.

Unless defined otherwise, all technical and scientific terms used herein have the same meanings as would generally understood by those skilled in the technical field of the present invention. The terms used herein in the specification of the present invention are for the purpose of describing specific embodiments only, and are not intended to limit the present invention. The term "and/or" used herein includes any and all combinations of one or more related listed items.

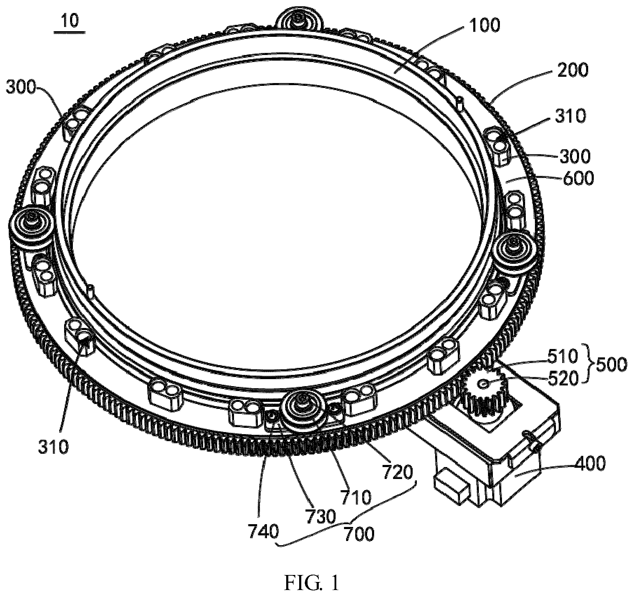

Referring to FIG. 1, an embodiment of the present invention provides a reaction cup conveying device 10.

The reaction cup conveying device 10 includes an inner circular conveying plate body 100, an outer circular conveying plate body 200, reaction cup loading assemblies 300, and a driving mechanism 400.

The outer circular conveying plate body 200 is sleeved on the inner circular conveying plate body 100 and can rotate relative to the inner circular conveying plate body 100. The plurality of reaction cup loading assemblies 300 are arranged on a plate surface of the outer circular conveying plate body 200 at intervals. The reaction cup loading assembly 300 is provided with an embedding groove 310 for embedding a reaction cup.

The driving mechanism 400 is connected to the outer circular conveying disc body 200 and is configured to drive the outer circular conveying disc body 200 to rotate. The driving mechanism 400 may be a driving motor.

In some embodiments, the reaction cup conveying device 10 further includes a conveying transmission mechanism 500. The conveying transmission mechanism 500 includes a transmission driving gear 510 and a transmission shaft 520. Gear teeth are provided on a periphery of the outer circular conveying plate body 200. The transmission driving gear 510 is connected to the driving mechanism 400 through the transmission shaft 520. The transmission driving gear 510 is engaged with the outer circular conveying plate body 200. The driving mechanism 400 drives the transmission driving gear 510 to rotate, so as to drive the outer circular conveying plate body 200 to rotate, thus conveying of reaction cups by the outer circular conveying plate body 200.

In some embodiments, a transmission ratio of the transmission driving gear 510 to the outer circular conveying plate body 200 ranges from 1:4 to 1:20. For example, in a specific embodiment, the transmission ratio of the transmission driving gear 510 to the outer circular conveying plate body 200 is 1:4. In another specific embodiment, the transmission ratio of the transmission driving gear 510 to the outer circular conveying plate body 200 is 1:10. In another specific embodiment, the transmission ratio of the transmission driving gear 510 to the outer circular conveying plate body 200 is 1:20. It is not difficult to understand that in other embodiments, the transmission ratio of the transmission driving gear 510 to the outer circular conveying plate body 200 may alternatively be 1:5, 1:6, 1:7, 1:8, 1:9, 1:11, 1:12, 1:12, 1:14, 1:15, 1:16, 1:17, 1:18, 1:19, or other non-integer ratios.

In some embodiments, the plurality of reaction cup loading assemblies 300 are distributed at intervals on the plate surface of the outer circular conveying plate body 200. The number of the plurality of reaction cup loading assemblies 300 on the plate surface of the outer circular conveying plate body 200 may be two, three, four, five, ..., ten, ..., fifteen, or like. Preferably, the plurality of reaction cup loading assemblies 300 on the plate surface of the outer circular conveying plate body 200 are evenly distributed.

In some embodiments, the outer circular conveying plate body 200 is a plastic plate body. A material of the outer circular conveying plate body 200 may be engineering plastic. The above reaction cup conveying device 10 realizes power transmission through the plastic outer circular conveying plate body 200 made of engineering plastic, and the engineering plastic has a low cost, which can reduce the overall cost of the reaction cup conveying device 10. It should be understood that in other embodiments, the above reaction cup conveying device 10 may alternatively use a ring metal gear to realize power transmission. Compared with the ring metal gear, the plastic outer circular conveying plate body 200 made of engineering plastic has a low cost.

In some embodiments, the reaction cup conveying device 10 further includes an outer plate reinforcing member 600. The outer plate reinforcing member 600 has a ring structure, and the outer plate reinforcing member 600 is connected to the outer circular conveying plate body 200. In order to enhance strength of the reaction cup conveying device 10, the reaction cup conveying device 10 is provided with the outer plate reinforcing member 600, so as to enhance structural strength of the outer circular conveying plate body 200. Through fitting between a metal sheet and the outer circular conveying plate body 200 made of engineering plastic, the structural strength of the outer circular conveying plate body 200 can be greatly improved, and stability of the conveying of the reaction cups is improved.

In some embodiments, the outer plate reinforcing member 600 is a metal sheet. A thickness of the outer plate reinforcing member 600 ranges from 0.1 mm to 10 mm. The outer circular conveying plate body 200 of the reaction cup conveying device 10 uses a structure of a gear made of engineering plastic and sleeved with a metal outer plate reinforcing member 600, which is light and simple in structure and has good gear strength and rigidity, stable power transmission, and durable and accurate positioning accuracy. In use, stability and accuracy of the reaction cup conveying device 10 are guaranteed.

For example, in an embodiment, the thickness of the outer plate reinforcing member 600 is 0.1 mm. In another embodiment, the thickness of the outer plate reinforcing member 600 is 5mm. In another embodiment, the thickness of the outer plate reinforcing member 600 is 10 mm. It should be understood that in other embodiments, the thickness of the outer plate reinforcing member 600 is 0.2 mm, 0.5 mm, 0.8 mm, 1 mm, 2 mm, 3 mm, 4 mm, 6 mm, 7 mm, 8 mm, 9 mm, or other values.

The reaction cup conveying device 10 further includes a sliding fit mechanism 700. The sliding fit mechanism 700 includes a fit rolling member 710, which is rotatably connected to the outer circular conveying plate body 200. The fit rolling member 710 abuts and rolls against an outer peripheral surface of the inner circular conveying plate body 100. In the reaction cup conveying device 10, through the arrangement of the fit rolling member 710, which is a V-shaped roller, an outer edge of the inner circular conveying plate body 100 can be lubricated, so that when rotating at a high speed, the outer circular conveying plate body 200 will not produce high friction noise, rub off powder, or even cause damages to and failure of the roller or the flange with the inner circular conveying plate body 100.

In some implementations not falling under the scope of the appended claims, the fit rolling member 710 may be a roller, a ball, a rolling ball, or the like. For example, in an embodiment, the fit rolling member 710 may be a roller, in another embodiment, the fit rolling member 710 may be a ball. In another embodiment, the fit rolling member 710 may be a rolling ball.

The sliding fit mechanism 700 further includes a rolling shaft 720. The fit rolling member 710 is a V-shaped roller. The V-shaped roller has a V-shaped groove on a rolling surface of the roller. In this way, a contact area between the fit rolling member 710 and the inner circular conveying plate body 100 can be reduced. The fit rolling member 710 is rotatably connected to the outer circular conveying plate body 200 through the rolling shaft 720. The V-shaped roller of the reaction cup conveying device 10 can lubricate a track groove through a structure of an oil storage tank and self-lubricating engineering plastics. It should be understood that in other embodiments, the V-shaped roller may alternatively be lubricated by regular maintenance. For example, lubricating oil is regularly applied to the V-shaped roller.

In some embodiments, an inner side of the fit rolling member 710 abuts against the outer peripheral surface of the inner circular conveying plate body 100, and an outer side of the fit rolling member 710 is coplanar with an edge of the outer circular conveying plate body 200.

In some embodiments, a plurality of sliding fit mechanisms 700 are provided. Through the arrangement of the plurality of sliding fit mechanisms 700, interaction between the inner circular conveying plate body 100 and the outer circular conveying plate body 200 can be achieved from a plurality of angles.

In some embodiments, the plurality of sliding fit mechanisms 700 are distributed at intervals on the outer circular conveying plate body 200.

In some embodiments, the plurality of sliding fit mechanisms 700 are distributed at equal intervals, that is, evenly distributed, on the outer circular conveying plate body 200. The sliding fit mechanisms 700 distributed at equal intervals can evenly exert forces on the inner circular conveying plate body 100, and realize contact and interaction between the inner circular conveying plate body 100 and the outer circular conveying plate body 200 in a plurality of directions.

In some embodiments, the sliding fit mechanism 700 further includes a bottom plate 730 and a fastener 740. The bottom plate 730 is connected to the outer plate reinforcing member 600 through the fastener 740. The fit rolling member 710 is connected to the bottom plate 730 through the rolling shaft 720. In the above reaction cup conveying device 10, through the arrangement of the bottom plate 730 and the fastener 740, a detachable connection between the fit rolling member 710 and the outer plate reinforcing member 600 can be achieved. By arranging detachable connection of the fit rolling member 710, operations such as disassembly, maintenance, and replacement thereof can be facilitated, and it is convenient for an operator to operate, thereby saving time and efforts.

This embodiment further provides an immunological detection apparatus.

An immunological detection apparatus includes the reaction cup conveying device 10.

The above reaction cup conveying device 10 has the following beneficial effects.
(1) The outer circular conveying plate body 200 of the reaction cup conveying device 10 uses a structure of a gear made of engineering plastic and sleeved with a metal outer plate reinforcing member 600, which is light and simple in structure and has good gear strength and rigidity, stable power transmission, and durable and accurate positioning accuracy. In use, stability and accuracy of the reaction cup conveying device 10 are guaranteed.
(2) In the reaction cup conveying device 10, through the arrangement of the fit rolling member 710 such as the V-shaped roller, the outer edge of the inner circular conveying plate body 100 can be lubricated, so that when rotating at a high speed, the outer circular conveying plate body 200 may not produce high friction noise, rub off powder, or even cause damages to and failure of the roller or the flange with the inner circular conveying plate body 100. Further, the V-shaped roller may use a self-lubricating material and an oil storage tank structure to prolong the overall service life of the device and reduce the cost of manual maintenance.
(3) The fit rolling member 710 such as the V-shaped roller is in contact with the inner circular conveying plate body 100 by abutment and tangency, i.e., point-to-point contact, which can minimize the contact area between the V-shaped roller and the inner circular conveying plate body 100, thereby reducing losses of mutual friction and also improving stability of the conveying of the outer circular conveying plate body 200 and positioning accuracy of the conveying. Point-to-point contact friction realizes relative motion, which makes the overall operation of the device more stable, so that the positioning accuracy is more accurate and adaptability is wider.
(4) In the reaction cup conveying device 10, through the positioning of the V-shaped roller, the outer circular conveying plate body 200 is suspended on the inner circular conveying plate body 100, the outer circular conveying plate body 200 and the inner circular conveying plate body 100 can convey the reaction cups independently, the outer circular conveying plate body 200 and the inner circular conveying plate body 100 can respectively meet system schemes under different time sequences, the outer circular conveying plate body 200 and the inner circular conveying plate body 100 can operate independently of each other, and positioning parameters can be associated with each other, so application scenarios are more extensive. In addition, the outer circular conveying plate body 200 and the inner circular conveying plate body 100 are positioned through the V-shaped roller, so that the whole device has a simple structure.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

The above embodiments only describe several implementations of the present invention, and their description is specific and detailed, but cannot therefore be understood as a limitation on the patent scope of the present invention. Therefore, the patent protection scope of the present invention should be subject to the appended claims.

## Claims

1. A reaction cup conveying device (10), comprising an inner circular conveying plate body (100), an outer circular conveying plate body (200), reaction cup loading assemblies (300), and a driving mechanism (400), wherein:
the outer circular conveying plate body (200) is sleeved on the inner circular conveying plate body (100) and is rotatable relative to the inner circular conveying plate body (100);
the plurality of reaction cup loading assemblies (300) are provided on a plate surface of the outer circular conveying plate body (200) at intervals;
the reaction cup loading assembly (300) is provided with an embedding groove (310) for embedding a reaction cup; and
the driving mechanism (400) is connected to the outer circular conveying disc body (200) and is configured to drive the outer circular conveying disc body (200) to rotate,
**characterized in that**:
the reaction cup conveying device (10) further comprises a sliding fit mechanism (700), wherein the sliding fit mechanism (700) comprises a fit rolling member (710) rotatably connected to the outer circular conveying plate body (200), and the fit rolling member (710) abuts and rolls against an outer peripheral surface of the inner circular conveying plate body (100),
wherein the sliding fit mechanism (700) further comprises a rolling shaft (720), the fit rolling member (710) is a V-shaped roller, and the fit rolling member (710) is rotatably connected to the outer circular conveying plate body (200) through the rolling shaft (720),
wherein the V-shaped roller has a V-shaped groove on a rolling surface of the roller, and
wherein the V-shaped roller is configured to lubricate an outer edge of the inner circular conveying plate body (100).

2. The reaction cup conveying device (10) according to claim 1, further comprising a conveying transmission mechanism (500), wherein the conveying transmission mechanism (500) comprises a transmission driving gear (510) and a transmission shaft (520), a periphery of the outer circular conveying plate body (200) is provided with gear teeth, the transmission driving gear (510) is connected to the driving mechanism (400) through the transmission shaft (520), and the transmission driving gear (510) is engaged with the outer circular conveying plate body (200).

3. The reaction cup conveying device (10) according to claim 2, wherein a transmission ratio of the transmission driving gear (510) to the outer circular conveying plate body (200) ranges from 1:4 to 1:20.

4. The reaction cup conveying device (10) according to any one of claim 1 to 3 further comprising an outer plate reinforcing member (600), wherein:
the outer plate reinforcing member (600) has a ring structure and is connected to the outer circular conveying plate body (200);
the plurality of reaction cup loading assemblies (300) are distributed at equal intervals on the plate surface of the outer circular conveying plate body (200); and/or
the outer circular conveying plate body (200) is a plastic plate body.

5. The reaction cup conveying device (10) according to claim 4, wherein the outer plate reinforcing member (600) is a metal sheet, and a thickness of the outer plate reinforcing member (600) ranges from 0.1 mm to 10 mm.

6. An immunological detection apparatus, comprising the reaction cup conveying device (10) according to any one of claims 1 to 5.

## Patentansprüche

1. Reaktionsgefäßbeförderungsvorrichtung (10), umfassend einen inneren kreisförmigen Förderplattenkörper (100), einen äußeren kreisförmigen Förderplattenkörper (200), Reaktionsgefäßladeanordnungen (300) und einen Antriebsmechanismus (400), wobei:
der äußere kreisförmige Förderplattenkörper (200) den inneren kreisförmigen Förderplattenkörper (100) ummantelt und relativ zu dem inneren kreisförmigen Förderplattenkörper (100) drehbar ist;
die Vielzahl von Reaktionsgefäßladeanordnungen (300) in Intervallen auf einer Plattenfläche des äußeren kreisförmigen Förderplattenkörpers (200) bereitgestellt ist;
die Reaktionsgefäßladeanordnung (300) mit einer Einbettungsrille (310) zum Einbetten eines Reaktionsgefäß bereitgestellt ist; und
der Antriebsmechanismus (400) mit dem äußeren kreisförmigen Förderplattenkörper (200) verbunden und dazu ausgelegt ist, den äußeren kreisförmigen Förderplattenkörper (200) in Drehung zu versetzen,
**dadurch gekennzeichnet, dass**:
die Reaktionsgefäßbeförderungsvorrichtung (10) ferner einen Gleitpassungsmechanismus (700) umfasst, wobei der Gleitpassungsmechanismus (700) ein Passwalzenelement (710) umfasst, das drehbar mit dem äußeren kreisförmigen Förderplattenkörper (200) verbunden ist, und wobei das Passwalzenelement (710) gegen eine Außenumfangsfläche des inneren kreisförmigen Förderplattenkörpers (100) anliegt und rollt,
wobei der Gleitpassungsmechanismus (700) ferner eine Walzenwelle (720) umfasst, wobei das Passwalzenelement (710) eine V-förmige Walze ist und das Passwalzenelement (710) über die Walzenwelle (720) drehbar mit dem äußeren kreisförmigen Förderplattenkörper (200) verbunden ist,
wobei die V-förmige Walze eine V-förmige Rille auf einer Walzenoberfläche der Walze aufweist und
wobei die V-förmige Rolle dazu ausgelegt ist, eine Außenkante des inneren kreisförmigen Förderplattenkörpers (100) zu schmieren.

2. Reaktionsgefäßbeförderungsvorrichtung (10) nach Anspruch 1, ferner umfassend einen Förderübertragungsmechanismus (500), wobei der Förderübertragungsmechanismus (500) ein Übertragungsantriebszahnrad (510) und eine Übertragungswelle (520) umfasst, wobei ein Umfang des äußeren kreisförmigen Förderplattenkörpers (200) mit Zahnrädern bereitgestellt ist, das Übertragungsantriebszahnrad (510) mit dem Antriebsmechanismus (400) über die Übertragungswelle (520) verbunden ist und das Übertragungsantriebszahnrad (510) mit dem äußeren kreisförmigen Förderplattenkörper (200) in Eingriff gebracht ist.

3. Reaktionsgefäßbeförderungsvorrichtung (10) nach Anspruch 2, wobei ein Übertragungsverhältnis des Übertragungsantriebszahnrads (510) zu dem äußeren kreisförmigen Förderplattenkörper (200) im Bereich von 1:4 bis 1:20 liegt.

4. Reaktionsgefäßbeförderungsvorrichtung (10) nach einem der Ansprüche 1 bis 3, ferner umfassend ein äußeres Plattenverstärkungselement (600), wobei:
das äußere Plattenverstärkungselement (600) eine Ringstruktur aufweist und mit dem äußeren kreisförmigen Förderplattenkörper (200) verbunden ist;
die Vielzahl von Reaktionsgefäßladeanordnungen (300) in regelmäßigen Intervallen auf der Plattenoberfläche des äußeren kreisförmigen Förderplattenkörpers (200) verteilt sind; und/oder
der äußere kreisförmige Förderplattenkörper (200) ein Kunststoffplattenkörper ist.

5. Reaktionsgefäßbeförderungsvorrichtung (10) nach Anspruch 4, wobei das äußere Plattenverstärkungselement (600) eine Metallfolie ist und eine Dicke des äußeren Plattenverstärkungselements (600) im Bereich von 0,1 mm bis 10 mm liegt.

6. Vorrichtung zur immunologischen Detektion, umfassend eine Reaktionsgefäßbeförderungsvorrichtung (10) nach einem der Ansprüche 1 bis 5.

## Revendications

1. Dispositif de transport de coupelle de réaction (10), comprenant un corps de plaque de transport circulaire interne (100), un corps de plaque de transport circulaire externe (200), des ensembles de chargement de coupelle de réaction (300), et un mécanisme d'entraînement (400), dans lequel :
le corps de plaque de transport circulaire externe (200) est emmanché sur le corps de plaque de transport circulaire interne (100) et peut tourner par rapport au corps de plaque de transport circulaire interne (100) ;
la pluralité d'ensembles de chargement de coupelle de réaction (300) sont prévus sur une surface de plaque du corps de plaque de transport circulaire externe (200) à des intervalles ;
l'ensemble de chargement de coupelle de réaction (300) est pourvu d'un évidement d'encastrement (310) pour encastrer une coupelle de réaction ; et
le mécanisme d'entraînement (400) est relié au corps de disque de transport circulaire externe (200) et est configuré pour entraîner le corps de disque de transport circulaire externe (200) en rotation,
**caractérisé en ce que** :
le dispositif de transport de coupelle de réaction (10) comprend en outre un mécanisme d'ajustement coulissant (700), dans lequel le mécanisme d'ajustement coulissant (700) comprend un élément de roulement d'ajustement (710) relié de manière rotative au corps de plaque de transport circulaire externe (200), et l'élément de roulement d'ajustement (710) vient en butée et roule contre une surface périphérique externe du corps de plaque de transport circulaire interne (100),
dans lequel le mécanisme d'ajustement coulissant (700) comprend en outre un arbre de roulement (720), l'élément de roulement d'ajustement (710) est un rouleau en forme de V, et l'élément de roulement d'ajustement (710) est relié de manière rotative au corps de plaque de transport circulaire externe (200) par l'intermédiaire de l'arbre de roulement (720),
dans lequel le rouleau en forme de V présente un évidement en forme de V sur une surface de roulement du rouleau, et
dans lequel le rouleau en forme de V est configuré pour lubrifier un bord interne du corps de plaque de transport circulaire interne (100).

2. Dispositif de transport de coupelle de réaction (10) selon la revendication 1, comprenant en outre un mécanisme de transmission de transport (500), dans lequel le mécanisme de transmission de transport (500) comprend un engrenage d'entraînement de transmission (510) et un arbre de transmission (520), une périphérie du corps de plaque de transport circulaire externe (200) est pourvue de dents d'engrenage, l'engrenage d'entraînement de transmission (510) est relié au mécanisme d'entraînement (400) par l'intermédiaire de l'arbre de transmission (520), et l'engrenage d'entraînement de transmission (510) est en prise avec le corps de plaque de transport circulaire externe (200).

3. Dispositif de transport de coupelle de réaction (10) selon la revendication 2, dans lequel un rapport de transmission de l'engrenage d'entraînement de transmission (510) au corps de plaque de transport circulaire externe (200) est compris entre 1:4 et 1:20.

4. Dispositif de transport de coupelle de réaction (10) selon l'une quelconque des revendications 1 à 3, comprenant en outre un élément de renforcement de plaque externe (600), dans lequel :
l'élément de renforcement de plaque externe (600) présente une structure annulaire et est relié au corps de plaque de transport circulaire externe (200) ;
la pluralité d'ensembles de chargement de coupelle de réaction (300) sont distribués à des intervalles égaux sur la surface de plaque du corps de plaque de transport circulaire externe (200) ; et/ou
le corps de plaque de transport circulaire externe (200) est un corps de plaque en plastique.

5. Dispositif de transport de coupelle de réaction (10) selon la revendication 4, dans lequel l'élément de renforcement de plaque externe (600) est une feuille métallique, et une épaisseur de l'élément de renforcement de plaque externe (600) est comprise entre 0,1 mm et 10 mm.

6. Appareil de détection immunologique, comprenant le dispositif de transport de coupelle de réaction (10) selon l'une quelconque des revendications 1 à 5.
